# EUROPEAN PATENT SPECIFICATION

(11) **EP 1 730 112 B1**
(45) Date of publication and mention of the grant of the patent: **03.09.2008**
(21) Application number: 05729157.7
(22) Date of filing: 24.03.2005
(51) Int. Cl.: C07D 215/24, C07D 401/12, C07D 413/12, A61K 31/4709, A61P 25/00

(54) **3-((HETERO)ARYLSULFONYL)-8'[ (AMINOALKYL)OXY]QUINOLINES AS 5-HT6 RECEPTOR ANTAGONISTS FOR THE TREATMENT OF CNS DISORDERS**
3-((HETERO)ARYLSULFONYL)-8-[(AMINOALKYL)OXY]CHINOLINE ALS ANTAGONISTEN DES 5-HT6-REZEPTORS ZUR BEHANDLUNG VON ERKRANKUNGEN DES ZNS
3-((HETERO)ARYLSULFONYL)-8'[ (AMINOALKYL)OXY]QUINOLINES SERVANT D'ANTAGONISTES AU RECEPTEUR 5-HT6 POUR LE TRAITEMENT DE TROUBLES du CNS

(30) Priority: 29.03.2004 GB 0407025
(43) Date of publication of application: 13.12.2006
(73) Proprietor: GLAXO GROUP LIMITED, Greenford, Middlesex UB6 ONN (GB)
(72) Inventor: AHMED, Mahmood GlaxoSmithKline, Harlow Essex CM19 5AW (GB); JOHNSON, Christopher, Norbert GlaxoSmithKline, Harlow Essex CM19 5AW (GB); MILLER, Neil, Derek GlaxoSmithKline, Harlow Essex CM19 5AW (GB); TRANI, Giancarlo GlaxoSmithKline, Harlow Essex CM19 5AW (GB); WITTY, David, R. GlaxoSmithKline, Harlow Essex CM19 5AW (GB)
(74) Representative: Billson, Siân Catherine
(86) International application number: PCT/GB2005/001106
(87) International publication number: WO 2005/095346

(56) References cited:
- EP-A- 1 188 747
- WO-A-02/100822
- WO-A-03/080580
- WO-A-03/080608
- WO-A-20/04000828
- WO-A-20/05021530
- WO-A-20/05026125

## Description

This invention relates to novel quinoline compounds having pharmacological activity, to processes for their preparation, to compositions containing them and to their use in the treatment of CNS and other disorders.

JP 02262627 (Japan Synthetic Rubber Co) describes a series of substituted quinoline derivatives useful as wavelength converting elements. WO 00/42026 (Novo Nordisk) describes a series of quinoline and quinoxaline compounds for use as GLP-1 agonists. WO 04/000828 (Biovitrum AB) describe a series of bicyclic sulfone or sulfonamide compounds which are claimed to be useful in the treatment or prophylaxis of a 5-HT₆ receptor related disorder. WO 00/71517 describes a series of phenoxypropylamine compounds as 5-HT_{1A} receptor antagonists which are claimed to be useful as antidepressants. WO 03/080608 and WO 03/080580 describe a series of quinoline compounds that have affinity for the 5-HT₆ receptor.

A structurally novel class of compounds has now been found which also possess antagonist potency for the 5-HT₆ receptor. The present invention therefore provides, in a first aspect, a compound of formula (I) or a pharmaceutically acceptable salt thereof: 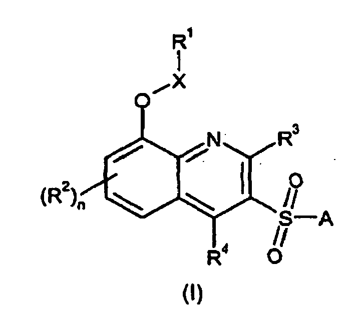 wherein:
R¹ represents a group of formula -NR^{a}R^{b} or a nitrogen containing heterocyclyl group optionally substituted by one or more (eg. 1 to 4) C₁₋₆ alkyl groups;
X represents a bond, -(CR^{c}R^{d})-, -(CR^{c}R^{d})-(CR^{e}R^{f})-. -(CR^{c}R^{d})-(CR^{e}R^{f})-(CR^{g}R^{h})- or-heterocyclyl-, wherein said heterocyclyl group may be optionally substituted by one or more (eg. 1 to 4) C₁₋₆ alkyl groups; such that when R¹ represents -NR^{a}R^{b}, X does not represent a bond nor -(CR^{c}R^{d})-;
R^{a}, R^{b}, R^{c}, R^{d}, R^{e}, R^{f}. R^{g} and R^{h} independently represent hydrogen or C₁₋₆ alkyl;
R² represents halogen, cyano, -CF₃, -CF₃O, C₁₋₆ alkyl, C₁₋₆ alkoxy, C₁₋₆ alkanoyl or a group -CONR⁵R⁶;
n represents 0 to 3;
R³ and R⁴ independently represent hydrogen, halogen, cyano, -CF₃, -CF₃O, C₁₋₆ alkyl, C₁₋₆ alkoxy, C₁₋₆ alkanoyl or a group -CONR⁵R⁶;
R⁵ and R⁶ Independently represent hydrogen or C₁₋₆ alkyl or together with the N atom to which they are attached may be fused to form a 5- to 7- membered nitrogen containing aromatic or non-aromatic heterocyclic ring optionally interrupted by an O or S atom;
A represents an -aryl, -heteroaryl, -aryl-aryl, -aryl-heteroaryl, -heteroaryl-aryl or - heteroaryl-heteroaryl group;
wherein said aryl and heteroaryl groups of A may be optionally substituted by one or more (eg. 1, 2 or 3) substituents which may be the same or different, and which are selected from the group consisting of halogen, hydroxy, cyano, nitro, trifluoromethyl, trifluoromethoxy, C₁₋₈ alkyl, trifluoromethanesulfonyloxy, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆, alkanoyl, C₁₋₆ alkoxycarbonyl, C₁₋₆ alkylsulfonyl, C₁₋₆ alkylsulfinyl, C₁₋₈ alkylsulfonyloxy, C₁₋₆ alkylsulfonylC₁₋₆ alkyl, arylsulfonyl, arylsulfonyloxy, arylsulfonylC₁₋₆ alkyl, C₁₋₆ alkylsulfonamido, C₁₋₆ alkylamido, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aroyl, aroylC₁₋₆ alkyl, arylC₁₋₆ alkanoyl, or a group CONR⁷R⁸ or SO₂NR⁷R⁸. wherein R⁷ and R⁸ independently represent hydrogen or C₁₋₆ alkyl or R⁷ and R⁸ together with the nitrogen atom to which they are attached may form a nitrogen containing heterocyclyl or heteroaryl group;
or solvates thereof.

Alkyl groups, whether alone or as part of another group, may be straight chain or branched and the groups alkoxy and alkanoyl shall be interpreted similarly. In one embodiment the alkyl moieties are C₁₋₄ alkyl, eg. methyl or ethyl.

The term 'cydoalkyl' means a closed 4- to 8- membered non-aromatic ring, for example cyclobutyl, cyclopentyl, cyclohexyl or cycloheptyl, or cyclooctyl.

The term 'halogen' is used herein to describe, unless otherwise stated, a group selected from fluorine, chlorine, bromine or iodine.

The term "aryl" includes single and fused rings for example, phenyl or naphthyl.

The term "heteroaryl" is intended to mean a 5-7 membered monocyclic aromatic or a fused 8-10 membered bicyclic aromatic ring containing 1 to 3 heteroatoms selected from oxygen, nitrogen and sulfur. Suitable examples of such monocyclic aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused bicyclic aromatic rings include quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like. Heteroaryl groups, as described above, may be linked to the remainder of the molecule via a carbon atom or, when present, a suitable nitrogen atom except where otherwise indicated above.

It will be appreciated that wherein the above mentioned aryl or heteroaryl groups have more than one substituent, said substituents may be linked to form a ring, for example a carboxyl and amine group may be linked to form an amide group.

The term "heterocyclyl" is intended to mean a 4-7 membered monocyclic saturated or partially unsaturated aliphatic ring containing 1 to 3 heteroatoms selected from oxygen, nitrogen or sulphur; a 4-7 membered monocyclic saturated or partially unsaturated aliphatic ring containing 1 to 3 heteroatoms selected from oxygen, nitrogen or sulphur fused to a benzene or monocyclic heteroaryl ring (referred to as fused rings); or an 8-membered bicyclic saturated nitrogen containing aliphatic ring. Suitable examples of such monocyclic rings include pyrrolidinyl, piperidinyl, piperazinyl, morpholinyl, thiamorpholinyl, diazepanyl, azepanyl, dihydroimidazolyl, tetrahydropyranyl, tetrahydrothiapyranyl and tetrahydrofuranyl. Suitable examples of fused rings include dihydroindolyl, dihydroisoindolyl, tetrahydroquinolinyl, tetrahydrobenzazepinyl and tetrahydroisoquinolinyl. A suitable example of an 8-membered bicyclic saturated nitrogen containing aliphatic ring is azabicyclo[2.2.2]octyl.

The term "nitrogen containing heterocyclyl" is intended to represent any heterocyclyl group as defined above which contains a nitrogen atom.

The term "nitrogen containing heteroaryl" is intended to represent any heteroaryl group as defined above which contains a nitrogen atom

The term "nitrogen containing non-aromatic heterocyclic ring" is intended to mean a 5 to 7 membered monocyclic saturated or partially unsaturated aliphatic ring containing 1 to 3 nitrogen atoms and further optionally interrupted by an oxygen or sulphur atom. Suitable examples of such rings include pyrrolidinyl, piparidinyl, piperazinyl, n-liorpholinyl, thiamorpholinyl, diazepanyl, azepanyl and dihydroimidazolyl.

The term "nitrogen containing aromatic heterocyclic ring" is intended to mean any 5 to 7 membered aromatic monocyclic ring containing 1 to 3 nitrogen atoms and further optionally interrupted by an oxygen or sulphur atom. Suitable examples of such rings include oxazolyl, thiazolyl, oxadiazolyl, isothiazolyl, isoxazolyl, thiadiazolyl, pyrazolyl, pyridyl, pyridazinyl, pyrimidinyl, pyrazinyl and triazinyl.

in one embodiment, R¹ represents a group of formula NR^{a}R^{b}, wherein R^{a} and R^{b} independently represent hydrogen or a methyl group.
In one embodiment, R¹ represents a 5- to 8-membered nitrogen containing heterocyclyl group optionally substituted by one or more C₁₋₃ alkyl groups.

In one embodiment, R¹ represents NR^{a}R^{b}, wherein R^{a} and R^{b} are independently hydrogen or methyl; or a nitrogen containing heterocyclyl group selected from pyrrolidinyl, piperidinyl, morpholinyl, azablcyclo[2.2.2]oct-3-yl or azepinyl any of which may be optionally substituted by methyl or isopropyl.
In one embodiment, X represents a bond, -(CR^{c}R^{d})-, -(CR^{c}R^{d})-(CR^{e}R^{f})-. -(CR^{c}R^{d})-(CR^{e}R^{f})-(CR^{g}R^{h})- or a tetrahydrofuranyl ring; such that when R¹ represents -NR^{a}R^{b}, X does not represent a bond nor-(CR^{c}R^{d})-.
In one embodiment, X represents a bond, -CH₂-, -CH₂-CH₂- or -C(H)(Me)-C(H)(Me)-.
In one embodiment, R¹-X- represents -(CH₂)₂-N(Me)₂, -CH₂-(1-methyl-2-pyrrolidinyl), - CH₂-(2-pyrrolidinyl), -(CH₂)₂-(1-pyrrolidinyl), -3-pyrrolidinyl, -C(H)(Me)-C(H)(Me)-N(Me)₂, -(CH₂)₂-(1-piperidinyl), -(CH₂)₂-(4-morpholinyl), -azabicyclo[2.2.2]oct-3-yl, - (CH₂)₃-(1-piperidinyl), -(CH₂)₂-(hexahydro-1H-azepin-1-yl), -4-amino-tetrahydro-3-furanyl), -4-dimethylamino-tetrahydro-3-furanyl, -3-piperidinyl, -4-piperidinyl, -1-methyl-3-pyrrolidinyl, -1-methyl-4-piperidinyl, -CH₂-(azabicyclo[2.2.2]oct-2-yl), -1-methyl-3-piperidinyl, -CH₂-(3-morpholinyl), -CH₂-(1-methytethyl-2-pyrrolidinyl) or -C(H)(Me)-CH₂-N(Me)₂.
In one embodiment, R¹-X- represents -(CH₂)₂-N(Me)₂, -CH₂-(1-methyl-2-pyrrolidinyl), - CH₂-(2-pyrrolidinyl), -(CH₂)₂-(1-pyrrolidinyl), -3-pyrrolidinyl or -C(H)(Me)-C(H)(Me)-N(Me)₂.
In one embodiment R^{a}, R^{b}, R^{c}, R^{d}, R^{e}, R^{f}, R^{g} and R^{h} independently represent hydrogen or a methyl group.
In one embodiment, R^{a} and R^{b} both represent C₁₋₆ alkyl (eg. methyl).
in one embodiment, R^{c} and R^{d} either both represent hydrogen or one represents hydrogen and the other represents C₁₋₆ alkyl (eg. methyl).
In one embodiment, R^{e} and R^{f} either both represent hydrogen or one represents hydrogen and the other represents C₁₋₆ alkyl (eg. methyl).
In one embodiment, n represents zero.
in one embodiment, n represents 1 and R² represents halogen.
In one embodiment, n represents 1 and R² represents chlorine.
In one embodiment, R³ and R⁴ both represent hydrogen.
In one embodiment, A represents -aryl (eg. phenyl) optionally substituted by one or more halogen (eg. chlorine) atoms or -heteroaryl (eg. pyridyl).
In a further embodiment, A represents -aryl (eg. phenyl) optionally substituted by a halogen (eg. chlorine).
In a further embodiment, A represents unsubstituted phenyl.
In yet a further embodiment there is provided a compound of formula (1a) or a pharmaceutically acceptable salt thereof: 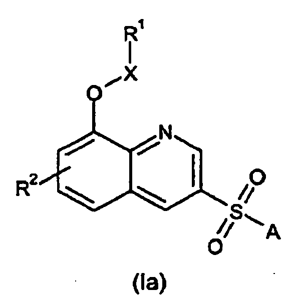 wherein:
R¹ represents a group of formula NR^{a}R^{b}, or a nitrogen containing heterocyclyl group selected from pyrrolidinyl, piperidinyl, morpholinyl, azablcyclo[2.2.2]oct-3-yl or azepinyl any of which may be optionally substituted by methyl or isopropyl;
X represents a bond, -(CR^{c}R^{d}, -(CR^{c}R^{d})-(CR^{e}R^{f})-, -(CR^{c}R^{d})-(CR^{e}R^{f})-(CR^{g}R^{h})- or a tetrahydrofuranyl ring; such that when R¹ represents -NR^{a}R^{b}, X does not represent a bond nor -(CR^{c}R^{d})-;
R^{a}, R^{b}, R^{c}, R^{d}, R^{e}, R^{f}, R^{g} and R^{h} independently represent hydrogen or a methyl group;
R² represents hydrogen or halogen; and
A represents phenyl optionally substituted by one or more halogen atoms.

Compounds according to the invention include examples E1-E27 as shown below, or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. The present invention includes within its scope all possible stoichiometric and non-stoichiometric forms.

The compounds of formula (I) may be prepared in crystalline or non-crystalline form, and, if crystalline, may optionally be solvated, eg. as the hydrate: This invention includes within its scope stoichiometric solvates (eg. hydrates) as well as compounds containing variable amounts of solvent (eg. water).

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) reacting a compound of formula (II) 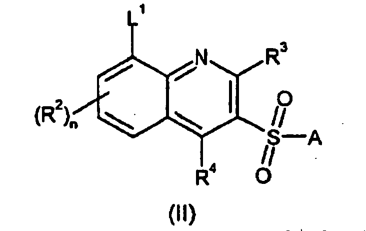 or an optionally protected derivative thereof, wherein R², R³, R⁴, n and A are as defined above and L¹ represents a leaving group such as a halogen atom or a trifluoromethylsulfonyloxy group;
   with a compound of formula R¹-X-OH or an optionally protected derivative thereof,
   wherein R¹ and X are as defined above, and optionally thereafter removing any protecting groups; or
(b) reacting a compound of formula (III) 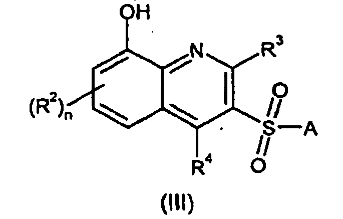 or an optionally protected derivative thereof; wherein R², R³, R⁴, n and A are as defined above;
   with a compound of formula R¹-X-L² or an optionally protected derivative thereof,
   wherein R¹ and X are as defined above and L² represents a leaving group such as a halogen atom or a methylsulfonyloxy group, and thereafter optionally removing any protecting groups; or
(c) reacting a compound of formula (III) as defined above or an optionally protected derivative thereof, with a compound of formula R¹-X-OH as defined above or an optionally protected derivative thereof, and thereafter optionally removing any protecting groups;
(d) deprotecting a compound of formula (I) which is protected;
(e) interconversion to other compounds of formula (I) and/or forming a pharmaceutically acceptable salt and/or solvate.

Process (a) typically comprises the use of basic conditions and may be conveniently carried out using a compound of formula (II) where L¹ represents a fluorine atom and an alkali metal salt of a compound of formula R¹-X-OH in a suitable solvent such as *N,N* dimethylformamide or dimethyl sulfoxide. The alkali metal salt of a compound of formula R¹-X-OH may be generated using a suitable alkali metal hydride such as sodium hydride. Alternatively, process (a) may be conveniently carried out using a compound of formula (II) where L¹ represents an iodine atom, in the presence of a base such as cesium carbonate and a suitable copper salt such as copper (I) iodide in a suitable solvent such as toluene. Process (a) may be optionally carried out at elevated temperature, e.g. 90 -110 °C.

Process (b) typically comprises the use of basic conditions and may be conveniently carried out either (i) using an alkali metal salt of a compound of formula (III), generated using a suitable alkali metal hydride such as sodium hydride, in a suitable solvent such as *N,N*-dimethylformamide or tetrahydrofuran or (ii) using a base such as potassium carbonate in a suitable solvent such as *N,N*-dimethylformamide, acetone or 2-butanone. Process (b) may be optionally carried out at elevated temperature, e.g. reflux temperature or 90 -110 °C.

Process (c) typically comprises the use of Mitsonobu conditions, using a suitable substituted phosphine such as triphenylphosphine and an appropriate azodicarbonyl reagent such as diethyl diazodicarboxylate in a suitable solvent such as dichloromethane or tetrahydrofuran.

In processes (a), (b), (c) and (d) examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid) or reductively (e.g. hydrogenolysis of a benzyl group or reductive removal of a 2',2',2'-trichloroethoxycarbony) group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid. A further amine protecting group includes methyl which may be removed using standard methods for N-dealkylation (e.g. 1-chloroethyl chloroformate under basic conditions followed by treatment with methanol).

Process (e) may be performed using conventional interconversion procedures such as epimerisation, oxidation, reduction, reductive alkylation, alkylation, nucleophilic or electrophilic aromatic substitution, ester hydrolysis or amide bond formation. For example, *N*-dealkylation of a compound of formula (I) wherein R^{a} represents an alkyl group to give a compound of formula (I) wherein R^{a} represents hydrogen. It will be appreciated that such interconversion may be interconversion of protected derivatives of formula (I) which may subsequently be deprotected following interconversion.

Compounds of formula (II) may be prepared as described in WO 03/080580.

Compounds of formula (II) wherein L¹ represents a fluorine or chlorine atom may be prepared by reacting a compound of formula (IV) 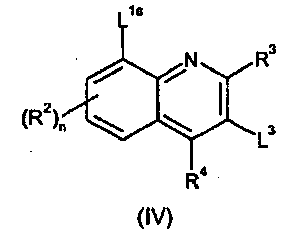 wherein L^{1a} is a fluorine or chlorine atom, L³ is a suitable leaving group such as an iodine atom, and R², R³, R⁴, and n are as defined above; with a compound of formula A-SO₂-M wherein A is as defined above and M is a metal residue such as sodium or potassium, in the presence of a copper (I) salt, e.g. copper (I) triflate or copper (I) iodide, in a suitable solvent such as dimethyl sulfoxide, anhydrous *N,N*-dimethylformamide or 1,4-dioxane, optionally including a ligand such as *N,N*'-dimethyl-ethylene-1,2-diamine and optionally in the presence of a base such as potassium carbonate.

Compounds of formula (III) may be prepared by reaction of a compound of formula (V) 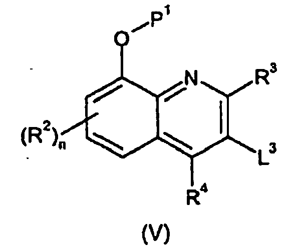 wherein L³, R², R³, R⁴, and n are as defined above and P¹ represents a suitable protecting group such as a trialkylsilylethyl group (e.g. trimethylsilylethyl) or a trifluoromethylsulfonyloxy group, with a compound of formula A-SO₂-M as defined above in a manner similar to that used to prepare compounds of formula (II); and thereafter removing the protecting group, eg. when P¹ represents a trialkylsilylethyl group, such deprotection may typically be carried out using an alkali metal fluoride salt or a tetraalkylammonium fluoride salt (eg. tetrabutylammonium fluoride).

Compounds of formula (IV) wherein L³ represents an iodine atom may be prepared by reacting a compound of formula (VI) 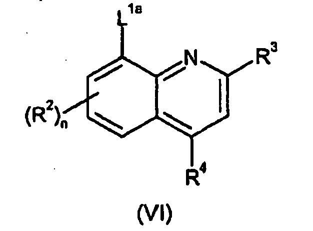 wherein L^{1a}, R², R³, R⁴ and n are as defined above; with an iodinating agent such as *N-*iodosuccinimide in a suitable solvent such as acetic acid.

Compounds of formula (V) wherein L³ represents an iodine atom may be prepared by reacting compounds of formula (VII) 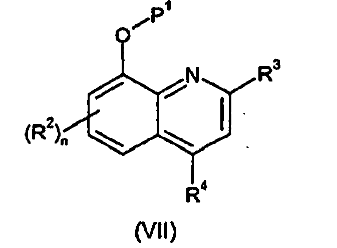 wherein R², R³, R⁴, n and P¹ are as defined above; with an iodinating agent such as *N-*iodosuccinimide in a suitable solvent such as acetic acid.

Compounds of formula (V) may also be prepared from compounds of formula (IV) as defined above by reaction with a compound of formula P¹-OH, wherein P¹ is as defined above, in the presence of a base such as sodium hydride in a suitable solvent such as tetrahydrofuran.

Compounds of formula (VI) and (VII) are either known in the literature or can be prepared by analogous methods.

Pharmaceutically acceptable salts may be prepared conventionally by reaction with the appropriate acid or acid derivative.

Compounds of formula (I) and their pharmaceutically acceptable salts have affinity for the 5-HT₆ receptor and are believed to be of potential use in the treatment of certain CNS disorders such as anxiety, depression, epilepsy, obsessive compulsive disorders, migraine, cognitive memory disorders (e.g. Alzheimers disease, age related cognitive decline, mild cognitive impairment and vascular dementia), Parkinsons Disease, ADHD (Attention Deficit Disorder/Hyperactivity Syndrome), sleep disorders (including disturbances of Circadian rhythm), feeding disorders such as anorexia and bulimia, panic attacks, withdrawal from drug abuse such as cocaine, ethanol, nicotine and benzodiazepines, schizophrenia (in particular cognitive deficits of schizophrenia), stroke and also disorders associated with spinal trauma and/or head injury such as hydrocephalus. Compounds of the invention are also expected to be of use in the treatment of certain GI (gastrointestinal) disorders such as IBS (Irritable Bowel Syndrome). Compounds of the invention are also expected to be of use in the treatment of obesity.

Thus the invention also provides a compound of formula (I) or a pharmaceutically acceptable salt thereof, for use as a therapeutic substance, in particular in the treatment or prophylaxis of the above disorders. In particular the invention provides for a compound of formula (I) or a pharmaceutically acceptable salt thereof, for use in the treatment of depression, anxiety, Alzheimers disease, age related cognitive decline, ADHD, obesity, mild cognitive impairment, schizophrenia, cognitive deficits in schizophrenia and stroke.

In another aspect, the invention provides the use of a compound of formula (I) or a pharmaceutical acceptable salt thereof in the manufacture of a medicament for use in the treatment or prophylaxis of the above disorders.

5-HT₆ antagonists have the potential to be capable of increasing basal and learning-induced polysialylated neuron cell frequency in brain regions such as the rat medial temporal lobe and associated hippocampus, as described in WO 03/066056. Thus, according to a further aspect of the present invention, we provide a method of promoting neuronal growth within the central nervous system of a mammal which comprises the step of administering a compound of formula (I) or a pharmaceutically acceptable salt thereof.

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended In the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

The following Descriptions and Examples illustrate the preparation of compounds of the invention.

### Description 1

### 8-Fluoro-3-iodoquinoline (D1)

*N*-Iodosuccinimide (8.1 g, 36.0 mmol, 2 eq.) was added to a solution of 8-fluoroquinoline (2.65 g, 18.0 mmol) in AcOH (13.25 ml, 5 vol). The mixture was stirred and placed in an oil bath which was then heated to 80°C. After 20 hrs 25min the flask was removed from the oil bath and allowed to cool to room temperature. CH₂Cl₂ (13.5 ml) was added, the solution was washed with 10% w/v Na₂SO_{3 (aq)} (23.5 ml), then with H₂O (13.5 ml) before being concentrated under reduced pressure. The crude product was pre-absorbed on silica and purified via column chromatography, eluting with 19:1 isohexane/EtOAc 1% Et₃N to yield 8-fluoro-3-iodoquinoline (D1) as a white solid (3.46 g, 12.7 mmol, 70%).
¹H NMR (CDCl₃, 400MHz) 8 7.40-7.45 (1H, m, Ar*H*). 7.50-7.52 (2H, m, Ar*H*). 8.58 (1H, t, *J* 1.7 Hz, Ar*H*), 9.09 (1H, d, *J* 2.0 Hz, Ar*H*).

### Description 2

### 8-Fluoro-3-(phenylsulfonyl)quinoline (D2)

A flask was charged with copper (I) iodide (70 mg, 0.366 mmol, 0.1eq.), 8-fluoro-3-iodoquinoline (D1) (1.00 g, 3.66 mmol), phenylsulfinic acid sodium salt (1.56 g, 10.98 mmol, 3 eq.) and potassium carbonate (1.01 g, 7.32 mmol, 2eq). DMSO (5 ml) then *N*, *N*'-dimethylethylene-1,4-diamine (0.078ml, 0.2 eq.) was added, the mixture was stirred and placed in an oil bath which was heated to 90 °C.
After heating for 3 ½ hrs the flask was removed from the oil bath and allowed to cool to room temperature. The mixture was filtered and the cake was washed with DMSO (2 x 2 ml), the cake was then slurried with water (4 ml) and filtered, then washed with water (2 x 2 ml), sucked dry and further dried in at 50 °C under reduced pressure to yield 8-fluoro-3-(phenylsulfonyl)quinoline (D2) as an off-white solid (0.485 g, 46%).
¹H NMR (CDCl₃, 400MHz) 8 7.54-7.67 (5H, m, Ar*H*). 7.78 (1 H, d, *J* 8.3 Hz, Ar*H*). 8.04 (2H, m, Ar*H*), 8.85 (1H, m, Ar*H*), 9.31 (1H, d, *J* 2.0 Hz, Ar*H)*.

### Example 1a

### [2-(3-Phenylsulfonylquinoline-8-yloxy)ethyl]dimethylamine (E1a)

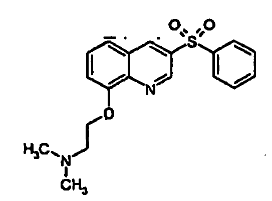

A round bottom flask was charged with copper (I) iodide (10 mg. 0.05 mmol), cesium carbonate (500 mg, 1.53 mmol), 2-dimethylaminoethanol (68 mg, 0.76 mmol) and 3-phenylsulfonyl-8-iodoquinoline (300 mg, 0.76 mmol) (WO 03/080580). The flask was then purged with argon and toluene (5 ml) introduced. The reaction mixture stirred was heated at reflux for 18 h. The reaction mixture was cooled and filtered. The filtrate was partitioned between dichloromethane (50 ml) and water (50 ml), the organic layer separated, dried over magnesium sulfate and concentrated to a brown paste. This was purified on silica, eluting with a dichloromethane/methanol (0 to 15%) gradient. [2-(3-Phenylsulfonylquinoline-8-yloxy)ethyl]dimethylamine (E1a) was obtained as a light brown solid (130 mg, 48%). ¹H NMR (CDCl₃) 8 2.40 (6H, s), 2.96 (2H, t, J = 6.0 Hz), 4.34 (2H, t, J = 6.2 Hz), 7.21-7.25 (2H, m), 7.51-7.56 (4H, m), 8.03 (2H, d, J = 7.1 Hz), 8.77 (1H, d, J = 2.0 Hz), 8.26 (1 H, br s).

### Example 1b

### [2-(3-Phenylsulfonylquinoline-8-yloxy)ethyl]dimethylamine, hydrochloride (E1b)

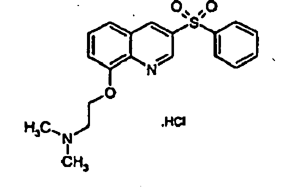

The free base was dissolved in methanol and transformed into the hydrochloride salt (E1b) by treating with HCl in Et₂O and stirring for 5 minutes followed by evaporation of the solvent MS: m/z (M+H)⁺ 357, C₁₉H₂₀N₂O₃S requires 356.

### Example 2a

### 8-({[(2S)-1-Methyl-2-pyrrolidinyl]methyl}oxy)-3-(phenylsulfonyl) quinoline (E2a)

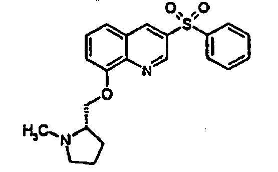

To a suspension of sodium hydride (60% dispersion in mineral oil) (50.4 mg, 1.26 mmol) in dry DMF (1.5 ml) in a pre-dried round bottomed flask was added [(2S)-1-methyl-2-pyrrolidinyl]methanol (0.15 ml, 1.26 mmol) under an argon atmosphere. The resulting mixture was stirred at 40 °C for five minutes. A solution of 8-fluoro-3-(phenylsulfonyl)quinoline (D2) (200 mg, 0.7 mmol) in dry DMF (2 ml) was added in one portion and the resulting mixture was stirred at 60 °C under argon for 15 hours. The mixture was applied to an Isolute Flash SCX column (5 g sorbent), washed with methanol, then the compound eluted with 10% ammonia in methanol. The residue was purified by flash chromatography (20 g silica gel) with a gradient of 10% methanolic ammonia in dichloromethane to give 8-({[(2*S*)-1-methyl-2-pyrrolidinyl]methyl}oxy)-3-(phenylsulfonyl)quinoline (E2a) as a yellow solid. ¹H NMR (CDCl₃) 8 9.27 (1H, d), 8.78 (1H, d), 8.00 (2H, dd), 7.55 (5H, m), 7.21 (1 H, dd), 4.23 (1H, dd), 4.08 (1H, dd), 3.14 (1H, dt), 2.93 (1H, m), 2.54 (3H, s), 2.33 (1H, m), 2.15 (2H, m), 1.85 (2H, m).

### Example 2b

### 8-({[(2S)-1-Methyl-2-pyrrolidinyl]methyl}oxy)-3-(phenylsulfonyl) quinoline, hydrochloride (E2b)

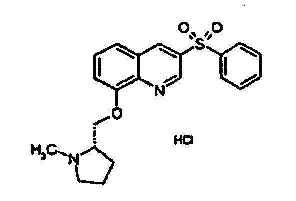

The free base was dissolved in methanol and transformed into the hydrochloride salt (E2b) by treating with HCl in Et₂O and stirring for 5 minutes followed by evaporation of the solvent. Mass spectrum: C₂₁H₂₂N₂O₃S requires 382; found 383 (MH⁺)

### Example 3

### 3-(Phenylsulfonyl)-8-{[(2S)-2-pyrrolidinylmethyl]oxy}quinoline, hydrochloride (E3)

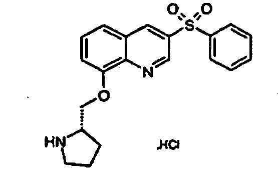

To a suspension of sodium hydride (60% dispersion in mineral oil) (36.4 mg, 0.91 mmol) in dry DMF (1.5 ml) in an oven dried round bottomed flask was added (2*S*)-2-pyrrolidinylmethanol (0.09 ml, 0.91 mmol) under argon atmosphere and the resulting mixture was stirred at 40 °C for five minutes. A solution of 8-fluoro-3-(phenylsulfonyl)quinoline (D2) (200 mg, 0.7 mmol) in dry DMF (2 ml) was added in one portion and the resulting mixture was stirred at 60 °C under argon over 15 hours. The mixture was applied to an Isolute Flash SCX column (5g sorbent), washed with methanol then eluted with 10% ammonia in methanol. The crude material was purified by flash chromatography (20 g silica gel) with a gradient of 10% methanolic ammonia in dichloromethane to afford 3-(phenylsulfonyl)-8-{[(2*S*-2-pyrrolidinylmethyl]-oxy}quinoline.
¹H NMR (CDCl₃) δ 9.26 (1H, d), 8.77 (1H, d), 8.02 (2H, dd), 7.54 (5H, m), 7.22 (1H, dd), 4.17 (1H, dd), 4.08 (1H, dd), 3.74 (1H, m), 3.01 (2H, m), 2.01 (1H, m), 1.86 (2H, m), 1.61 (1H, m).
This material was dissolved in methanol and transformed into the hydrochloride salt (E3) a yellow solid, by treating with HCl (1 M in Et₂O, 0.7 ml) and stirring for 5 minutes followed by evaporation of the solvent.
Mass spectrum: C₂₀H₂₀N₂O₃S requires 368; found 369 (MH⁺)

### Example 4

### 3-(Phenylsulfonyl)-8-{[2-(1-pyrrolidinyl)ethyl]oxy}quinoline, hydrochloride (E4)

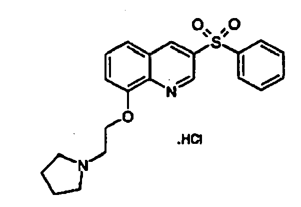

This compound was prepared in a similar manner to the methods of Example E1a and Example E1b but using 2-(1-pyrrolidinyl)ethanol in place of 2-dimethylaminoethanol, affording 3-(phenylsulfonyl)-8-{[2-(1-pyrrolidinyl)ethyl]oxy}quinoline, hydrochloride (E4). Mass spectrum: C₂₁H₂₂N₂O₃S requires 382; Found 383 (MH⁺)

### Example 5

### 3-(Phenylsulfonyl)-8-[(3R)-3-pyrrolidinyloxy]quinoline, hydrochloride (E5)

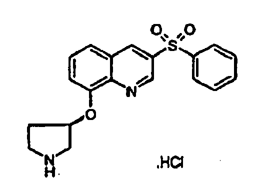

A suspension of sodium hydride (60% oil dispersion, 43mg) in DMF (2mL), was treated dropwise with a solution of 1,1-dimethylethyl (3*R*)-3-hydroxy-1-pyrrolidinecarboxylate (1.81 mmol, 338 mg) in DMF (3 mL) and the mixture stirred for 10 minutes. The resulting solution was treated with 8-fluoro-3-(phenylsulfonyl)quinoline (D2) (400 mg, 1.39 mmol) in DMF (5 mL). The mixture was heated to 60°C for 16h, then cooled, poured into dichloromethane (70 mL), washed with water (50 mL) then brine (50 mL), dried (MgSO₄) and evaporated to afford a brown oil (764 mg). This was purified by silica gel chromatography (50 g cartridge) eluting with a linear gradient of increasing ethyl acetate in pentane to give 1,1-dimethylethyl (3*R*)-3-{[3-(phenylsulfonyl)-8-quinolinyl]oxy}-1-pyrrolidinecarboxylate (426 mg). This was treated with hydrogen chloride in 1,4-dioxane (4M, 5 mL) for two hours then the solvent removed *in vacuo.* The residue was further purified by preparative reverse phase chromatography, and the resulting material by normal phase chromatography on silica gel eluting with dichloromethane and an increasing gradient of 10% aqueous ammonia in methanol. The resulting solid was treated with methanol (2 mL) and hydrogen chloride in diethyl ether (1M, 1 mL) followed by evaporation to give 3-(phenylsulfonyl)-8-[(3*R*)-3-pyrrolidinyloxy]quinoline, hydrochloride (E5).
Mass spectrum: C₁₉H₁₈N₂O₃S requires 354; found 355 (MH⁺)

### Examples 6-13 (E6-E13)

Examples 6-13 were prepared in a similar manner to the procedure described in Example 1. or Example 3, from the corresponding hydroxyalkylamine indicated in the table below:

| **Example** | **Structure** | **Hydroxyalkyl -amine** | **Compound Name** | **Analogous Method to** | **Mass Spectrum** |
|---|---|---|---|---|---|
| **E6** | 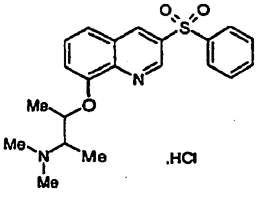 | 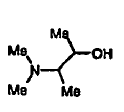 | Dimethyl(1-methyl-2-{[3-(phenylsulfonyl)-8-quinolinyl]oxy} propyl)amine, hydrochloride | E3 | Requires 384; Found 385 (MH⁺) |
| **E7** | 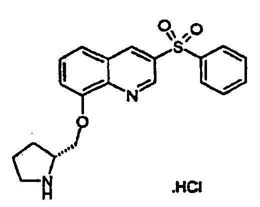 | 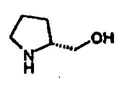 | 3-(Phenylsulfonyl)-8-{[(2*R*)-2-pyrrolidinylmethyl] oxy}quinoline. hydrochloride | E3 | Requires 354; Found 355 (MH⁺) |
| **E8** | 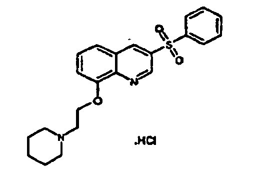 | 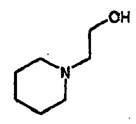 | 3-(Phenylsulfonyl)-8-{[2-(1-piperidinyl)ethyl]oxy} quinoline, hydrochloride | E1 | Requires 396; Found 397 (MH⁺) |
| **E9** | 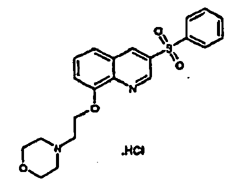 | 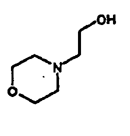 | 8-{[2-(4-Morpholinyl)ethyl]ox y}-3-(phenylsulfonyl) quinoline, hydrochloride | E1 | Requires 398; Found 399 (MH⁺) |
| **E10** | 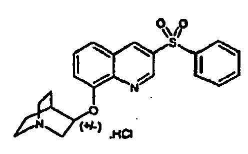 | 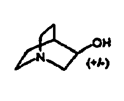 | 8-(1-Azabicyclo[2.2.2]oct -3-yloxy)-3-(phenylsulfonyl) quinoline, hydrochloride | E3 | Requires 394; Found . 395 (MH⁺) |
| **E11** | 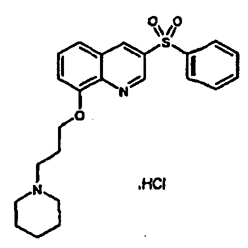 | 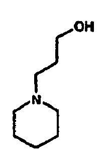 | 3-(Phenylsulfonyl)-8-{[3-(1-piperidinyl)propyl] oxy}quinoline. hydrochloride | E3 | Requires 410; Found 411 (MH⁺) |
| **E12** | 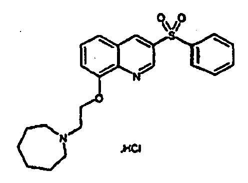 | 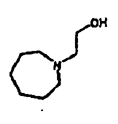 | 8-{[2-(Hexahydro-1H-azepin-1-yl)ethyl]oxy}-3-(phenylsulfonyl) quinoline, hydrochloride | E3 | Requires 410; Found 411 (MH⁺) |
| **E13** | 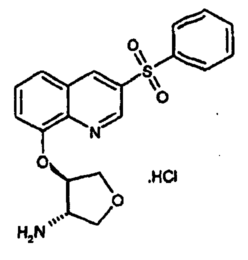 | 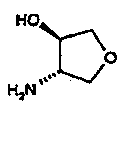 | ((3S,4R)-4-{[3-(Phenylsulfonyl)-8-quinolinyl]oxy} tetrahydro-3-furanyl)amine, hydrochloride | E3 | Requires 370; Found 371 (MH⁺) |

### Example 14

### (35,4R)-N,N-Dimethyl-4-{[3-(phenylsulfonyl)-8-quinolinyl]oxy}tetrahydro-3-furanamine, hydrochloride (E14)

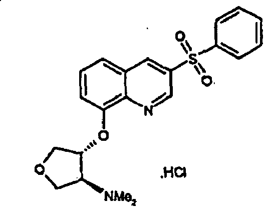

The free base form of ((3S,4R)-4-{[3-(phenylsulfonyl)-8-quinolinyl]oxy} tetrahydro-3-furanyl)amine, from Example E13 (60 mg) was treated with formalin (37% formaldehyde in water, 0.2 mL), in 1,2-dichloroethane (3 mL) and sodium triacetoxyborohydride (137 mg) added. The mixture was stirred for 18 hours then filtered through an SCX cartridge (10g), eluting with methanol then 10% aqueous ammonia in methanol to afford a yellow paste. This was treated with hydrogen chloride in methanol (1M), to give, after evaporation, (3S,4R)-N,N-dimethyl-4-{[3-(phenylsulfonyl)-8-quinolinyl]oxy}tetrahydro-3-furanamine, hydrochloride (E14)
Mass spectrum: C₂₁H₂₂N₂O₄S requires 398; Found 399 (MH⁺)

### Examples 15-26 (E15-E26)

Examples 15-26 were prepared either in a similar manner to the procedure described in Example 3 from the corresponding hydroxyalkylamines in the table below, or in a similar manner to the procedure described in Example 14 using the carbonyl compounds and parent amines from Example numbers indicated in the table below.

| **Example** | **Structure** | **Hydroxyalkylamine** | **Carbonyl Compound and Parent Amine Example** | **Compound Name** | **Analogous Method to** | **Mass Spectrum** |
|---|---|---|---|---|---|---|
| **E15** | 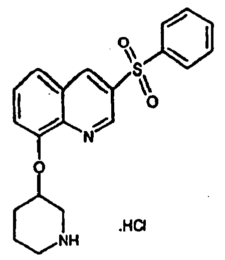 | 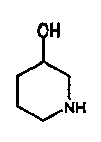 | - | 3-(Phenylsulfonyl)-8-(3-piperidinyloxy) quinoline, hydrochloride | E3 | Requires 368; Found 369 (MH⁺) |
| **E16** | 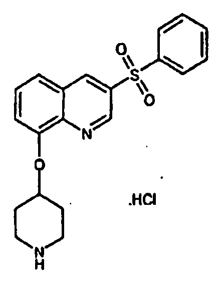 | 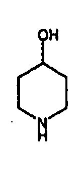 | - | 3-(Phenylsulfonyl)-8-(4-piperidinyloxy) quinoline, hydrochloride | E3 | Requires 368; Found 369 (MH⁺) |
| **E17** | 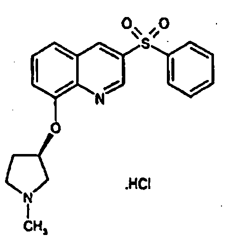 | 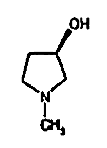 | - | 8-{[(3R)-1-Methyl-3-pyrrolidinyl]oxy)-3-(phenylsulfonyl) quinoline, hydrochloride | E3 | Requires 368; Found 369 (MH⁺⁾ |
| **E18** | 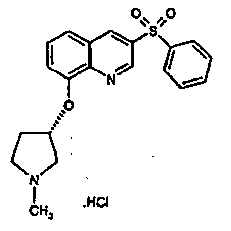 | 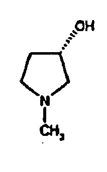 | - | 8-{[(3S)-1-Methyl-3-pyrrolidinyl]oxy}-3-(phenylsulfonyl) quinoline, hydrochloride | E3 | Requires 368; Found 369 (MH⁺) |
| **E19** | 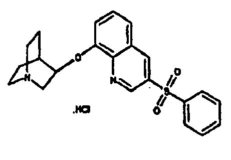 | 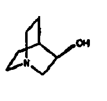 | - | 8-[(3R)-1-Azabicyclo[2.2.2]oct-3-yloxy]-3-(phenylsulfonyl) quinoline, hydrochloride | E3 | Requires 394; Found 395 (MH⁺) |
| **E20** | 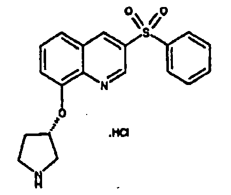 | 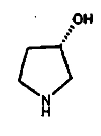 | - | 3-(Phenylsulfonyl)-8-[(3S)-3-pyrrolidinyloxy] quinoline, hydrochloride | E3 | * |
| **E21** | 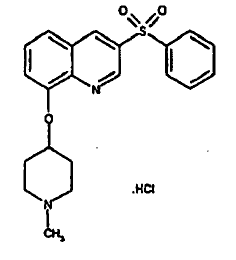 | - | Formalin E16E16 | 8-[(1-Methyl-4-piperidinyl)oxy]-3- (phenylsulfonyl) quinoline, hydrochloride | E14 | Requires 382; Found 383 (MH⁺) |
| **E22** | 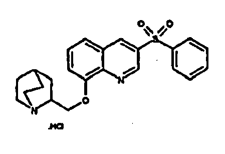 | 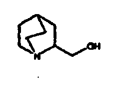 | - | 8-[(1-Azabicyclo[22.2]oct-2-ylmethyl)oxy]-3-(phenylsulfonyl) quinoline, hydrochloride | E3 | Requires 408; Found 409 (MH⁺) |
| **E23** | 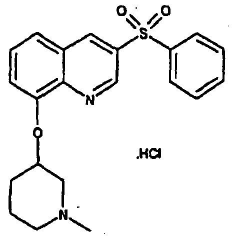 | - | Formalin E15 | 8-[(1-Methyl-3-piperidinyl)oxy]-3-(phenylsulfonyl) quinoline, hydrochloride | E14 | Requires 382; Found 383 (MH⁺) |
| **E24** | 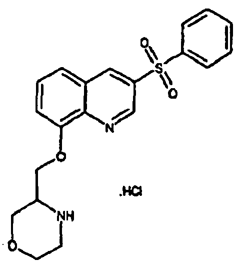 | 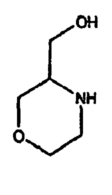 | - | 8-[(3-Morpholinylmethyl) oxy]-3-(phenylsulfonyl) quinoline, hydrochloride | E3 | Requires 384; Found 385 (MH⁺) |
| **E25** | 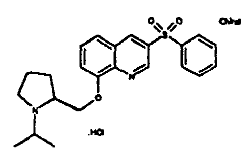 | - | Acetone E3 | 8-({[(2S)-1-(1-Methylethyl-2-pyrrolidinyl]methyl) oxy)-3-(phenylsulfonyl) quinoline, hydrochloride | E14 | Requires 410; Found 411 (MH⁺) |
| **E26** | 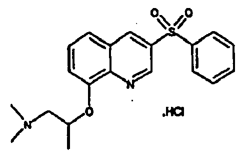 | 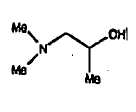 | - | N,N-Dimethyl-2-{(3-(phenylsulfonyl)-8-quinolinyl]oxy)-1-propanamine, hydrochloride | E3 | Requires 370; Found 371 (MH⁺) |

| | | | | | | |
|---|---|---|---|---|---|---|
| ***For E20:** ¹H NMR (CD₃OD): 2.30-2.45 (m, 2H), 3.67-3.73 (m, 1H), 3.84-3.92 (m, 2H), 4.02-4.15 (m, 1H), 5.50-5.54 (m, 1H), 7.64-7.72 (m, 2H), 7.85 (d, J = 7Hz, 1H), 7.95-8.05 (m, 2H), 8.10-8.20 (m, 2H), 8.23 (d, J = 9Hz, 1H), 9.45 (br s, 1H), 9.76 (br s, 1H). | | | | | | |

### Example 27

### 5-Chloro-3-(phenylsulfonyl)-8-2-{[2-(1-pyrrolidinyl)ethyl]oxy}quinoline hydrochloride (E27)

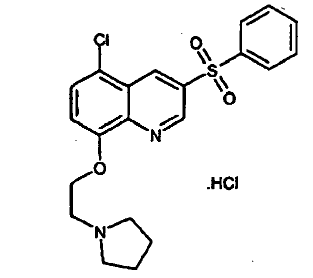

To a stirred solution of 3-(phenylsulfonyl)-8-{[2-(1-pyrrolidinyl)ethyl]oxy}quinoline (0.1g. 0.26mmol) in acetic acid (2mL) was added N-chlorosuccinimide (38mg, 0.29mmol) in a single portion and the resulting mixture heated to 100°C for 18h. After allowing to cool to ambient temperature, the reaction mixture was concentrated *in vacuo* followed by purification by preparative HPLC. The resulting material was treated with 1 M HCl/E₂O to afford the title compound as a beige solid (35mg). Mass spectrum: C₂₁H₂₁N₂O₃SCl Requires 416/418; Found 417/419 (MH*)

### Pharmacological data

Compounds of the invention may be tested for *in vitro* biological activity in accordance with the following cyclase assay:

### Cyclase Assay

0.5µl of test compound in 100% dimethylsulfoxide (DMSO) was added to a white, solid 384 well assay plate (for dose response measurements the top of the concentration range is 7.5µM final). 10µl of washed membranes of HeLa 5HT₈ cells (for preparation see WO 98/27081) in basic buffer (50mM HEPES pH 7.4 (KOH), 10mM MgCl₂, 100mM NaCl, 1µl/ml 3-isobutyl-1-methylxanthine (IBMX) (Sigma-Aldrich)) was added to all wells followed by 10µl 2 x ATP buffer (100µl/ml ATP and 1µl/ml 3-Isobutyl-1-methylxanthine (IBMX) (Sigma-Aldrich)) with 5-HT (at a concentration equivalent to a dose response of 4 x EC₅₀). The resultant mixture was then incubated at room temperature for 30-45 minutes to allow cAMP production.

cAMP production was then measured using the DiscoveRx^{™}HitHunter^{™} chemiluminescence cAMP assay kit (DiscoveRx Corporation, 42501 Albrae Street, Fremont, CA 94538; Product Code: 90-0004L) or any other suitable cAMP measurement assay.

IC₅₀ values were estimated from arbitrary designated unit (ADU) measurements from a Perkin Elmer Viewlux instrument using a four parameter logistic curve fit within EXCEL (Bowen, W.P. and Jerman, J.C. (1995), Nonlinear regression using spreadsheets. Trends in Pharmacol. Sci., 16, 413-417). Functional Kᵢ values were calculated using the method of Cheng, Y.C. and Prussof, W.H. (Biochemical Pharmacol (1973) 22 3099-3108). plC₅₀ and fpKᵢ are the negative log10 of the molar IC₅₀ and functional Kᵢ respectively.

The compounds of Examples E1b, E2b and E3-E27, were tested in the above cyclase assay. Compounds of Examples E1b, E2b, E3-E7, E15, E17 and E22-E24 showed antagonist potency for the 5-HT₆ receptor, having fpKi values > 8.0 at human cloned 5-HT₆ receptors. The compounds of all other Examples also showed antagonist potency for the 5-HT₆ receptor, having fpKi values ≥ 7.0 and < 8.0 at human cloned 5-HT₆ receptors.

## Claims

1. A compound of formula (I) or a pharmaceutically acceptable salt thereof: 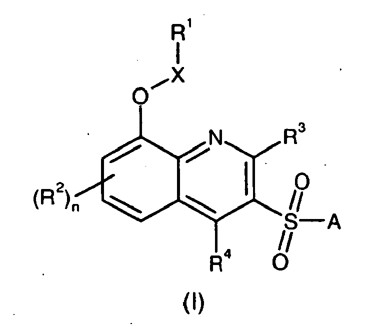 wherein:
R¹ represents a group of formula -NR^{a}R^{b} or a nitrogen containing heterocyclyl group optionally substituted by one or more (eg. 1 to 4) C₁₋₆ alkyl groups;
X represents a bond, -(CR^{c}R^{d})-, -(CR^{c}R^{d})-(CR^{e}R^{f})-, -(CR^{c}R^{d})-(CR^{e}R^{f})-(CR^{g}R^{h})-, or - heterocyclyl-, wherein said heterocyclyl group may be optionally substituted by one or more (eg. 1 to 4) C₁₋₆ alkyl groups; such that when R¹ represents -NR^{a}R^{b}, X does not represent a bond nor -(CR^{c}R^{d})-;
R^{a}, R^{b}, R^{c}, R^{d}, R^{e}, R^{f}, R^{g} and R^{h} independently represent hydrogen or C₁₋₆ alkyl;
R² represents halogen, cyano, -CF₃, -CF₃O, C₁₋₆ alkyl, C₁₋₅ alkoxy, C₁₋₆ alkanoyl or a group -CONR⁵R⁶;
n represents 0 to 3;
R³ and R⁴ independently represent hydrogen, halogen, cyano, -CF₃, -CF₃O, C₁₋₆ alkyl, C₁₋₆ alkoxy, C₁₋₆ alkanoyl or a group -CONR⁵R⁶;
R⁵ and R⁶ independently represent hydrogen or C₁₋₆ alkyl or together with the N atom to which they are attached may be fused to form a 5- to 7- membered N-containing aromatic or non-aromatic heterocyclic ring optionally interrupted by an O or S atom; A represents an -aryl, -heteroaryl, -aryl-aryl, -aryl-heteroaryl, -heteroaryl-aryl or - heteroaryl-heteroaryl group;
wherein said aryl and heteroaryl groups of A may be optionally substituted by one or more (eg. 1, 2 or 3) substituents which may be the same or different, and which are selected from the group consisting of halogen, hydroxy, cyano, nitro, trifluoromethyl, trifluoromethoxy, C₁₋₆ alkyl, trifluoromethanesulfonyloxy, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, arylsulfonyl, arylsulfonyloxy, arylsulfonylC₁₋₆ alkyl, C₁₋₆ alkylsulfonamido, C₁₋₆ alkylamido, C₁₋₆ alkylsulfonamidoC₁₋₅ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aroyl, aroylC₁₋₆ alkyl, arylC₁₋₆ alkanoyl, or a group CON⁷R⁸ or SO₂NR⁷R⁸, wherein R⁷ and R⁸ independently represent hydrogen or C₁₋₆ alkyl or R⁷ and R⁸ together with the nitrogen atom to which they are attached may form a nitrogen containing heterocyclyl or heteroaryl group.

2. A compound of formula (I) as defined in claim 1, wherein A represents aryl optionally substituted by one or more halogen atoms or heteroaryl.

3. A compound of formula (I) as defined in claim 1 or claim 2, wherein R¹ represents NR^{a}R^{b}, wherein R^{a} and R^{b} are independently hydrogen or methyl; or a nitrogen containing heterocyclyl group selected from pyrrolidinyl, piperidinyl, morpholinyl, azabicyclo[2.2.2]oct-3-yl or azepinyl optionally substituted by methyl or isopropyl.

4. A compound of formula (I) as defined in claim 1 selected from:
[2-(3-Phenylsulfonylquinoline-8-yloxy)ethyl]dimethylamine;
8-({[(2S)-1-Methyl-2-pyrrolidinyl]methyl}oxy)-3-(phenylsulfonyl) quinoline;
3-(Phenylsulfonyl)-8-{[(2*S*)-2-pyrrolidinylmethyl]oxy}quinoline;
3-(Phenylsulfonyl)-8-{[2-(1-pyrrolidinyl)ethyl]oxy}quinoline;
3-(Phenylsulfonyl)-8-[(3*R*)-3-pyrrolidinyloxy]quinoline;
Dimethyl(1-methyl-2-{[3-(phenylsulfonyl)-8-quinolinyl]oxy} propyl)amine;
3-(Phenylsulfonyl)-8-{[(2*R*)-2-pyrrolidinylmethyl]oxy}quinoline;
3-(Phenylsulfonyl)-8-{[2-(1-piperidinyl)ethyl]oxy}quinoline;
8-{[2-(4-Morpholinyl)ethyl]oxy}-3-(phenylsulfonyl)quinoline;
8-(1-Azabicyclo[2.2.2]oct-3-yloxy)-3-(phenylsulfonyl)quinoline;
3-(Phenylsulfonyl)-8-{[3-(1-piperidinyl)propyl]oxy}quinoline;
8-{[2-(Hexahydro-1H-azepin-1-yl)ethyl]oxy}-3-(phenylsulfonyl)quinoline;
((3S,4R)-4-4{[3-(Phenylsulfonyl)-8-quinolinyl]oxy}tetrahydro-3-furanyl)amine;
(3S,4R)-N,N-Dimethyl-4-{[3-(phenylsulfonyl)-8-quinolinyl]oxy}tetrahydro-3-furanamine;
3-(Phenylsulfonyl)-8-(3-piperidinyloxy)quinoline;
3-(Phenylsulfonyl)-8-(4-piperidinyloxy)quinoline;
8-{[(3R)-1-Methyl-3-pyrrolidinyl]oxy}-3-(phenylsulfonyl)quinoline;
8-{[(3S)-1-Methyl-3-pyrrolidinyl]oxy}-3-(phenylsulfonyl)quinoline;
8-[(3R)-1-Azabicyclo[2.2.2]oct-3-yloxy]-3-(phenylsulfonyl)quinoline;
3-(Phenylsulfonyl)-8-[(3S)-3-pyrrolidinyloxy]quinoline;
8-[(1-Methyl-4-piperidinyl)oxy]-3-(phenylsulfonyl)quinoline;
8-[(1-Azabicyclo[2.2.2]oct-2-ylmethyl)oxy]-3-(phenylsulfonyl)quinoline;
8-[(1-Methy)-3-piperidinyl)oxy]-3-(pheny)sulfonyl)quinoline;
8-[(3-Morpholinylmethyl)oxy]-3-(phenylsulfonyl)quinoline;
8-({[(2S)-1-(1-Methylethyl)-2-pyrrolidinyl]methyl}oxy)-3-(phenylsulfonyl)quinoline;
N,N-Dimethyl-2-{[3-(phenylsulfonyl)-8-quinolinyl]oxy}-1-propanamine; or
5-Chloro-3-(phenylsulfonyl)-8-{[2-(1-pyrrolidinyl)ethyl]oxy}quinoline;
or a pharmaceutically acceptable salt thereof.

5. A compound of claim 4 wherein the salt is the hydrochloride salt.

6. A pharmaceutical composition which comprises a compound or a pharmaceutically acceptable salt as defined in any one of claims 1 to 5 and a pharmaceutically acceptable carrier or excipient.

7. A compound or pharmaceutically acceptable salt as defined in any one of claims 1 to 5 for use in the treatment of anxiety, depression, epilepsy, obsessive compulsive disorders, migraine, cognitive memory disorders Parkinsons Disease ADHD (Attention Deficit Disorder/Hyperactivity Syndrome), sleep disorders, feeding disorders, panic attacks, withdrawal from drug abuse, schizophrenia, stroke and also disorders associated with spinal trauma and/or head injury, Irritable Bowel Syndrome and obesity,

8. A compound or pharmaceutically acceptable salt as defined in any one of claims 1 to 5 for use in the treatment of depression, anxiety, Alzheimers disease, age related cognitive decline. ADHD, obesity, mild cognitive impairment, schizophrenia, cognitive deficits in schizophrenia and stroke.

9. The use of a compound of formula (I) or a pharmaceutically acceptable saltthereof as defined in any one of claims 1 to 5 in the manufacture of a medicament for the treatment or prophylaxis of anxiety, depression, epilepsy, obsessive compulsive disorders migraine, cognitive memory disorders, Parkinsons Disease, ADHD (Attention Deficit Disorder/Hyperactivity Syndrome), sleep disorders, feeding disorders, panic attacks, withdrawal from drug abuse, schizophrenia, stroke and also disorders associated with spinal trauma and/or head injury, Irritable Bowel Syndrome and obesity

10. The use of a compound of formula (I) or a pharmaceutically acceptable salt thereof as defined in any one of claims 1 to 5 in the manufacture of a medicament for the treatment or prophylaxis of depression, anxiety, Alzheimers disease, age related cognitive decline, ADHD, obesity, mild cognitive impairment, schizophrenia, cognitive deficits in schizophrenia and stroke.

11. A pharmaceutical composition comprising a compound of formula (I) or apharmaceutically acceptable salt thereof as defined in any one of claims 1 to 5 for use in the treatment of anxiety, depression, epilepsy, obsessive compulsive disorders. migraine, cognitive memory disorders, Parkinsons Disease, ADHD (Attention Deficit Disorder/Hyperactivity Syndrome), sleep disorders, feeding disorders, panic attacks, withdrawal from drug abuse, schizophrenia, stroke and also disorders associated with spinal trauma and/or head injury, Irritable Bowel Syndrome and obesity.

12. A pharmaceutical composition comprising a compound of formula (I) or a pharmaceutically acceptable salt thereof as defined in any one of claims 1 to 5 for use in the treatment of depression, anxiety, Alzheimers disease, age related cognitive decline. ADHD, obesity, mild cognitive Impairment, schizophrenia, cognitive deficits in schizophrenia and stroke.

## Patentansprüche

1. Verbindung der Formel (I) oder ein pharmazeutisch verträgliches Salz davon: 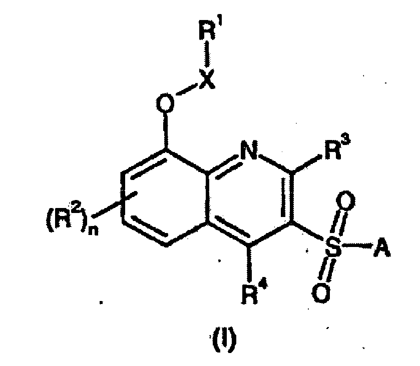 wobei:
R¹ ein Rest der Formel -NR^{a}R^{b} oder ein Stickstoff enthaltender Heterocyclylrest, gegebenenfalls substituiert durch einen oder mehrere (z. B. 1 bis 4) C₁₋₆-Alkylreste, ist;
X eine Bindung, -(CR^{c}R^{d})-, -(CR^{c}R^{d})-(CR^{e}R^{f})-, -(CR^{c}R^{d})-(CR^{e}R^{f})-(CR^{g}R^{h})-, oder -Heterocyclyl- ist, wobei der Heterocyclylrest gegebenenfalls durch einen oder mehrere (z. B. 1 bis 4) C₁₋₆-Alkylreste substituiert sein kann; derart dass, wenn R¹ -NR^{a}R^{b} ist, X weder eine Bindung noch -(CR^{c}R^{d})- ist;
R^{a}, R^{b}, R^{c}, R^{d}, R^{e}, R^{f}, R^{g} und R^{h} unabhängig voneinander Wasserstoff oder C₁₋₆-Alkyl sind;
R² Halogen, Cyano, -CF₃, -CF₃O, C₁₋₆-Alkyl, C₁₋₆-Alkoxy, C₁₋₆-Alkanoyl oder ein Rest -CONR⁵R⁶ ist;
n gleich 0 bis 3 ist;
R³ und R⁴ unabhängig voneinander Wasserstoff, Halogen, Cyano, -CF₃, -CF₃O, C₁₋₆-Alkyl, C₁₋₆-Alkoxy, C₁₋₆-Alkanoyl oder ein Rest -CONR⁵R⁶ sind;
R⁵ und R⁶ unabhängig voneinander Wasserstoff oder C₁₋₆-Alkyl oder zusammen mit dem Stickstoffatom, an welches sie gebunden sind, aniliert sein können, um einen, gegebenenfalls durch ein O- oder S-Atom unterbrochenen 5- bis 7-gliedrigen N-enthaltenden aromatischen oder nicht-aromatischen heterocyclischen Ring zu bilden; A ein -Aryl, -Heteroaryl, -Aryl-Aryl, -Aryl-Heteroaryl, -Heteroaryl-Aryl oder -Heteroaryl-Heteroarylrest ist;
wobei die Aryl- und Heteroarylreste von A gegebenenfalls durch eine oder mehrere (z. B. 1, 2 oder 3) Substituenten, welche gleich oder verschieden sein können und welche ausgewählt sind aus Halogen, Hydroxy, Cyano, Nitro, Trifluormethyl, Trifluormethoxy, C₁₋₆-Alkyl, Trifluormethansulfonyloxy, Pentafluorethyl, C₁₋₆-Alkoxy, ArylC₁₋₆-Alkoxy, C₁₋₆-Alkylthio, C₁₋₆-AlkoxyC₁₋₆-Alkyl, C₃₋₇-CycloalkylC₁₋₆-Alkoxy, C₁₋₆-Alkanoyl, C₁₋₆-Alkoxycarbonyl, C₁₋₆-Alkylsulfonyl, C₁₋₆-Alkylsulfinyl, C₁₋₆-Alkylsulfonyloxy, C₁₋₆-AlkylsulfonylC₁₋₆-Alkyl, Arylsulfonyl, Arylsulfonyloxy, ArylsulfonylC₁₋₆-Alkyl, C₁₋₆-Alkylsulfonamido, C₁₋₆-Alkylamido, C₁-₆-AlkylsulfonamidC₁₋₆-Alkyl, C₁₋₆-AlkylamidoC₁₋₆-Alkyl, Arylsulfonamido, Arylcarboxamido, ArylsulfonamidoC₁₋₆-Alkyl, ArylcarboxamidoC₁₋₆-Alkyl, Aroyl, AroylC₁₋₆-Alkyl, ArylC₁₋₆-Alkanoyl oder einen Rest CONR⁷R⁸ oder SO₂NR⁷R⁸ substituiert sein können, wobei R⁷ und R⁸ unabhängig voneinander Wasserstoff oder C₁₋₆-Alkyl sind oder R⁷ und R⁸ zusammen mit dem Stickstoffatom, an welches sie gebunden sind, einen Stickstoff enthaltenden Heterocyclyl- oder Heteroarylrest bilden können.

2. Verbindung der Formel (I) wie in Anspruch 1 definiert, wobei A Aryl, gegebenenfalls mit einem oder mehreren Halogenatomen oder Heteroaryl substituiert, ist.

3. Verbindung der Formel (I) wie in Anspruch 1 oder Anspruch 2 definiert, wobei R¹ NR^{a}R^{b}, wobei R^{a} und R^{b} unabhängig voneinander Wasserstoff oder Methyl sind; oder ein Stickstoff-enthaltender Heterocyclylrest, ausgewählt aus Pyrrolidinyl, Piperidinyl, Morpholinyl, Azabicyclo[2.2.2]oct-3-yl oder Azepinyl, gegebenenfalls durch Methyl oder Isopropyl substituiert, ist.

4. Verbindung der Formel (I) wie in Anspruch 1 definiert, ausgewählt aus:
[2-(3-Phenylsulfonylchinolin-8-yloxy)ethyl]dimethylamin;
8-({[(2*S*)-1-Methyl-2-pyrrolidinyl]methyl}oxy)-3-(phenylsulfonyl)chinolin;
3-(Phenylsulfonyl)-8-{[(2*S*)-2-pyrrolidinylmethyl]oxy}chinolin;
3-(Phenylsulfonyl)-8-{[(2-(1-pyrrolidinyl)ethyl]oxy}chinolin;
3-(Phenylsulfonyl)-8-[(3*R*)-3-pyrrolidinyloxy]chinolin;
Dimethyl(1-methyl-2-{[3-(phenylsulfonyl)-8-chinolinyl]oxy}propyl)amin;
3-(Phenylsulfonyl)-8-{([(2*R*)-2-pyrrolidinylmethyl]oxy}chinolin;
3-(Phenylsulfonyl)-8-{[2-(1-piperidinyl)ethyl]oxy}chinolin;
8-{[2-(4-Morpholinyl)ethyl]oxy}-3-(phenylsulfonyl)chinolin;
8-(1-Azabicyclo[2.2.2]oct-3-yloxy)-3-(phenylsulfonyl)chinolin;
3-(Phenylsulfonyl)-8-{[3-(1-piperidinyl)propyl]oxy}chinolin;
8-{[2-(Hexahydro-1H-azepin-1-yl)ethyl]oxy}-3-(phenylsulfonyl)chinolin;
((3*S*,4*R*)-4-{[3-(Phenylsulfonyl)-8-chinolinyl]oxy}tetrahydro-3-furanyl)amin;
(3*S*,4*R*)-N,N-Dimethyl-4-{[3-(phenylsulfonyl)-8-chinolinyl]oxy}tetrahydro-3-furanamin;
3-(Phenylsulfonyl)-8-(3-piperidinyloxy)chinolin;
3-(Phenylsulfonyl)-8-(4-piperidinyloxy)chinolin;
8-{[(3*R*)-1-Methyl-3-pyrrolidinyl]oxy}-3-(phenylsulfonyl)chinolin;
8-{[(3*S*)-1-Methyl-3-pyrrolidinyl]oxy}-3-(phenylsulfonyl)chinolin;
8-[(3*R*)-1-Azabicyclo[2.2.2]oct-3-yloxy]-3-(phenylsulfonyl)chinolin;
3-(Phenylsulfonyl)-8-[(3S)-3-pyrrolidinyloxy]chinolin;
8-[(1-Methyl-4-piperidinyl)oxy]-3-(phenylsulfonyl)chinolin;
8-[(1-Azabicyclo[2.2.2]oct-2-ylmethyl)oxy]-3-(phenylsulfonyl)chinolin;
8-[(1-Methyl-3-piperidinyl)oxy]-3-(phenylsulfonyl)chinolin;
8-[(3-Morpholinylmethyl)oxy]-3-(phenylsulfonyl)chinolin;
8-({[(2*S*)-1-(1-Methylethyl)-2-pyrrolidinyl]methyl}oxy)-3-(phenylsulfonyl)chinolin;
N,N-Dimethyl-2-{[3-(phenylsulfonyl)-8-chinolinyl]oxy}-1-propanamin; oder
5-Chlor-3-(phenylsulfonyl)-8-{[2-(1-pyrrolidinyl)ethyl]oxy}chinolin;
oder ein pharmazeutisch verträgliches Salz davon.

5. Verbindung nach Anspruch 4, wobei das Salz das Hydrochlorid ist.

6. Arzneimittel, welches eine Verbindung oder ein pharmazeutisch verträgliches Salz wie in einem der Ansprüche 1 bis 5 definiert und einen pharmazeutisch verträglichen Träger oder Hilfsstoff umfasst.

7. Verbindung oder pharmazeutisch verträgliches Salz wie in einem der Ansprüche 1 bis 5 definiert, zur Verwendung bei der Behandlung von Angst, Depression, Epilepsie, Zwangsstörungen, Migräne, kognitiven Gedächtnisstörungen, Parkinson-Krankheit, ADHD (Aufmerksamkeitsdefizits-Syndrom/Hyperaktivitätssyndrom), Schlafstörungen, Fütterstörung, Panikattacken, Drogenmissbrauchsentzugserscheinungen, Schizophrenie, Schlaganfall und auch Störungen in Verbindung mit spinalem Trauma und/oder Kopfverletzungen, Reizdarmsyndrom und Fettleibigkeit.

8. Verbindung oder pharmazeutisch verträgliches Salz wie in einem der Ansprüche 1 bis 5 definiert, zur Verwendung bei der Behandlung von Depression, Angst, Alzheimer-Krankheit, altersbedingtem kognitiven Abbau, ADHD, Fettleibigkeit, leichter kognitiver Störung, Schizophrenie, kognitiven Defiziten bei Schizophrenie und Schlaganfall.

9. Verwendung einer Verbindung der Formel (I) oder eines pharmazeutisch verträglichen Salzes davon, wie in einem der Ansprüche 1 bis 5 definiert, zur Herstellung eines Medikaments zur Behandlung oder Prophylaxe von Angst, Depression, Epilepsie, Zwangsstörungen, Migräne, kognitiven Gedächtnisstörungen, Parkinson-Krankheit, ADHD (Aufmerksamkeitsdefizits-Syndrom/Hyperaktivitätssyndrom), Schlafstörungen, Fütterstörung, Panikattacken, Drogenmissbrauchsentzugserscheinungen, Schizophrenie, Schlaganfall und auch Störungen in Verbindung mit spinalem Trauma und/oder Kopfverletzungen, Reizdarmsyndrom und Fettleibigkeit.

10. Verwendung einer Verbindung der Formel (I) oder eines pharmazeutisch verträglichen Salzes davon, wie in einem der Ansprüche 1 bis 5 definiert, zur Herstellung eines Medikaments zur Behandlung oder Prophylaxe von Depression, Angst, Alzheimer-Krankheit, altersbedingtem kognitiven Abbau, ADHD, Fettleibigkeit, leichter kognitiver Störung, Schizophrenie, kognitiven Defiziten bei Schizophrenie und Schlaganfall.

11. Arzneimittel, umfassend eine Verbindung der Formel (I) oder ein pharmazeutisch verträgliches Salzes davon, wie in einem der Ansprüche 1 bis 5 definiert, zur Verwendung bei der Behandlung von Angst, Depression, Epilepsie, Zwangsstörungen, Migräne, kognitiven Gedächtnisstörungen, Parkinson-Krankheit, ADHD (Aufmerksamkeitsdefizits-Syndrom/Hyperaktivitätssyndrom), Schlafstörungen, Fütterstörung, Panikattacken, Drogenmissbrauchsentzugserscheinungen, Schizophrenie, Schlaganfall und auch Störungen in Verbindung mit spinalem Trauma und/oder Kopfverletzungen, Reizdarmsyndrom und Fettleibigkeit.

12. Arzneimittel, umfassend eine Verbindung der Formel (I) oder ein pharmazeutisch verträgliches Salz davon, wie in einem der Ansprüche 1 bis 5 definiert, zur Verwendung bei der Behandlung von Depression, Angst, Alzheimer-Krankheit, altersbedingten kognitiven Abbau, ADHD, Fettleibigkeit, leichter kognitiver Störung, Schizophrenie, kognitiven Defiziten bei Schizophrenie und Schlaganfall.

## Revendications

1. Composé de formule (I) ou un de ses sels pharmaceutiquement acceptables : 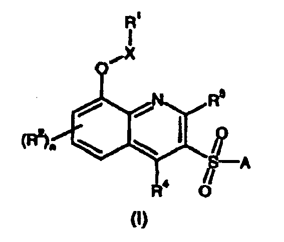 formule dans laquelle :
R¹ représente un groupe de formule -NR^{a}R^{b} ou un groupe hétérocyclyle azoté facultativement substitué avec un ou plusieurs (par exemple 1 à 4) groupes alkyle en C₁ à C₆ ;
X représente une liaison, un groupe - (CR^{c}R^{d})- , - (CR^{c}R^{d}) - (CR^{e}R^{f}) - , - (CR^{c}R^{d}) - (CR^{e}R^{f}) - (CR^{g}R^{h}) - ou -hétérocyclyle- , ledit groupe hétérocyclyle pouvant être facultativement substitué avec un ou plusieurs (par exemple 1 à 4) groupes alkyle en C₁ à C₆ ; de telle sorte que, lorsque R¹ représente un groupe -NR^{a}R^{b}, X ne représente ni une liaison ni un groupe -(CR^{c}R^{d}) - ;
R^{a}, R^{b}, R^{c}, R^{d}, R^{e}, R^{f}, R^{g} et R^{h} représentent indépendamment un atome d'hydrogène ou un groupe alkyle en C₁ à C₆ ;
R² représente un groupe halogéno, cyano, -CF₃, -CF₃O, alkyle en C₁ à C₆, alkoxy en C₁ à C₆, alcanoyle en C₁ à C₆ ou un groupe -CONR⁵R⁶ ;
n a une valeur de 0 à 3 ;
R³ et R⁴ représentent indépendamment un atome d'hydrogène, un groupe halogéno, cyano, -CF₃, -CF₃O, alkyle en C₁ à C₆, alkoxy en C₁ à C₆, alcanoyle en C₁ à C₆ ou un groupe -CONR⁵R⁶ ;
R⁵ et R⁶ représentent indépendamment un atome d'hydrogène ou un groupe alkyle en C₁ à C₆ ou bien, conjointement avec l'atome de N auquel ils sont fixés, ils peuvent être condensés pour former un noyau hétérocyclique aromatique ou non aromatique azoté penta- à heptagonal facultativement interrompu par un atome de O ou S ;
A représente un groupe -aryle, -hétéroaryle, -arylaryle, -aryl-hétéroaryle, -hétéroaryl-aryle ou -hétéroarylhétéroaryle ;
lesdits groupes aryle et hétéroaryle de A pouvant être facultativement substitués avec un ou plusieurs (par exemple 1, 2 ou 3) substituants qui peuvent être identiques ou différents et qui sont choisis dans le groupe consistant en des substituants halogéno, hydroxy, cyano, nitro, trifluorométhyle, trifluorométhoxy, alkyle en C₁ à C₆, trifluorométhanesulfonyloxy, pentafluoréthyle, alkoxy en C₁ à C₆, aryl(alkoxy en C₁ à C₆), alkylthio en C₁ à C₆, (alkoxy en C₁ à C₆) - (alkyle en C₁ à C₆) , (cycloalkyle en C₃ à C₇) (alkoxy en C₁ à C₆) , alcanoyle en C₁ à C₆, (alkoxy en C₁ à C₆) carbonyle, alkylsulfonyle en C₁ à C₆, alkylsulfinyle en C₁ à C₆, alkylsulfonyloxy en C₁ à C₆, (alkyle en C₁ à C₆) - sulfonyl (alkyle en C₁ à C₆) , arylsulfonyle, arylsulfonyloxy, arylsulfonyl(alkyle en C₁ à C₆), (alkyle en C₁ à C₆) sulfonamido, alkylamido en C₁ à C₆, (alkyle en C₁ à C₆) - sulfonamido (alkyle en C₁ à C₆) , (alkyle en C₁ à C₆) amido-(alkyle en C₁ à C₆), arylsulfonamido, arylcarboxamido, arylsulfonamido(alkyle en C₁ à C₆), arylcarboxamido(alkyle en C₁ à C₆) , aroyle, aroyl (alkyle en C₁ à C₆) , aryl (alcanoyle en C₁ à C₆) ou un groupe CONR⁷R⁸ ou SO₂NR⁷R⁸, dans lequel R⁷ et R⁸ représentent indépendamment un atome d'hydrogène ou un groupe alkyle en C₁ à C₆ ou bien R⁷ et R⁸, conjointement avec l'atome d'azote auquel ils sont fixés, peuvent former un groupe hétérocyclyle ou hétéroaryle azoté.

2. Composé de formule (I) suivant la revendication 1, dans lequel A représente un groupe aryle facultativement substitué avec un ou plusieurs atomes d'halogènes ou hétéroaryle.

3. Composé de formule (I) suivant la revendication 1 ou la revendication 2, dans lequel R¹ représente un groupe NR^{a}R^{b}, dans lequel R^{a} et R^{b} représentent indépendamment un atome d'hydrogène ou un groupe méthyle ; ou un groupe hétérocyclyle azoté choisi entre des groupes pyrrolidinyle, pipéridinyle, morpholinyle, azabicyclo[2.2.2]oct-3-yle ou azépinyle facultativement substitué avec un substituant méthyle ou isopropyle.

4. Composé de formule (I) suivant la revendication 1, choisi entre :
la [2- (3-phénylsulfonylquinoléine-8-yloxy) éthyl] - diméthylamine ;
la 8-({[(2*S*)-1-méthyl-2-pyrrolidinyl]méthyl}oxy)-3-(phénylsulfonyl) quinoléine ;
la 3-(phénylsulfonyl)-8-{[(2*S*)-2-pyrrolidinylméthyl]-oxy}quinoléine ;
la 3-(phénylsulfonyl)-8-{[2-(1-pyrrolidinyl)éthyl]-oxy}quinoléine ;
la 3-(phénylsulfonyl)-8-[(3*R*)-3-pyrrolidinyloxy]-quinoléine;
la diméthyl(1-méthyl-2-{ [3-(phénylsulfonyl) -8-quinolinyl]-oxy}propyl) amine ;
la 3- (phénylsulfonyl) -8- {[(2*R*) -2-pyrrolidinylméthyl]-oxy}quinoléine ;
la 3-(phénylsulfonyl) -8-{[2- (1-pipéridinyl)éthyl]oxy}-quinoléine ;
la 8-{ [2- (4-morpholinyl) éthyl] oxy} -3- (phénylsulfonyl) - quinoléine ;
la 8-(1-azabicyclo[2.2.2]oct-3-yloxy)-3-(phénylsulfonyl)-quinoléine ;
la 3-(phénylsulfonyl)-8-{[3-(1-pipéridinyl)propyl]-oxy}quinoléine ;
la 8- { [2- (hexahydro-1H-azépine-1-yl) éthyl] oxy}-3-(phénylsulfonyl)quinoléine ;
la ((3S,4R)-4-{[3-(phénylsulfonyl)-8-quinolinyl]oxy}-tétrahydro- 3 - furannyl ) amine ;
la (3S, 4R) -N,N-diméthyl-4-{ [3- (phénylsulfonyl) -8-quinolinyl] - oxy}tétrahydro-3-furanamine ;
la 3-(phénylsulfonyl)-8-(3-pipéridinyloxy)quinoléine ;
la 3-(phénylsulfonyl)-8-(4-pipéridinyloxy)quinoléine ;
la 8-{[(3R)-1-méthyl-3-pyrrolidinyl]oxy}-3-(phénylsulfonyl)-quinoléine ;
la 8-{[(3S)-1-méthyl-3-pyrrolidinyl]oxy}-3-(phénylsulfonyl)-quinoléine ;
la 8-{[(3R)-1-azabicyclo[2.2.2]oct-3-yloxy]-3-(phénylsulfonyl)quinoléine ;
la 3- (phénylsulfonyl) -8- [ (3S) -3-pyrrolidinyloxy] - quinoléine ;
la 8-[(1-méthyl-4-pipéridinyl)oxy]-3-(phénylsulfonyl)-quinoléine ;
la 8-[(1-azabicyclo[2.2.2]oct-2-ylméthyl)oxy]-3-(phénylsulfonyl)quinoléine ;
la 8- [ (1-méthyl-3-pyrrolidinyl) oxy] -3- (phénylsulfonyl) - quinoléine ;
la 8- [ (3-morpholinylméthyl ) oxy] -3- (phénylsulfonyl) - quinoléine ;
la 8-({[(2S)-1-(1-méthyléthyl)-2-pyrrolidinyl]méthyl}-oxy)-3-(phénylsulfonyl)quinoléine ;
la N,N-diméthyl-2-{ [3- (phénylsulfonyl) -8-quinolinyl] - oxy}-1-propanamine ;
la 5-chloro-3-(phénylsulfonyl)-8-{[2-(1-pyrrolidinyl)-éthyl]oxy}quinoléine ;
ou un de ses sels pharmaceutiquement acceptables.

5. Composé suivant la revendication 4, dans lequel le sel est le chlorhydrate.

6. Composition pharmaceutique qui comprend un composé ou un sel pharmaceutiquement acceptable suivant l'une quelconque des revendications 1 à 5, et un support ou excipient pharmaceutiquement acceptable.

7. Composé ou sel pharmaceutiquement acceptable suivant l'une quelconque des revendications 1 à 5, destiné à être utilisé dans le traitement de l'anxiété, de la dépression, de l'épilepsie, de troubles obsessionnels compulsifs, de la migraine, de troubles cognitifs de la mémoire, de la maladie de Parkinson, du ADHD (trouble de déficit d'attention/syndrome d'hyperactivité), de troubles du sommeil, de troubles de l'alimentation, d'attaques de panique, du sevrage après l'abus de substances médicamenteuses, de la schizophrénie, d'un ictus et également de troubles associés à un traumatisme rachidien et/ou une lésion crânienne, du syndrome du côlon irritable et de l'obésité.

8. Composé ou sel pharmaceutiquement acceptable suivant l'une quelconque des revendications 1 à 5, destiné à être utilisé dans le traitement de la dépression, de l'anxiété, de la maladie d'Alzheimer, du déclin cognitif dû à l'âge, du ADHD, de l'obésité, de l'altération cognitive faible, de la schizophrénie, de déficits cognitifs dans la schizophrénie et d'un ictus.

9. Utilisation d'un composé de formule (I) ou d'un de ses sels pharmaceutiquement acceptables suivant l'une quelconque des revendications 1 à 5, dans la production d'un médicament destiné au traitement ou à la prophylaxie de l'anxiété, de la dépression, de l'épilepsie, de troubles obsessionnels compulsifs, de la migraine, de troubles cognitifs de la mémoire, de la maladie de Parkinson, du ADHD (trouble de déficit d'attention/syndrome d'hyperactivité), de troubles du sommeil, de troubles de l'alimentation, d'attaques de panique, du sevrage après l'abus de substances médicamenteuses, de la schizophrénie, d'un ictus et également de troubles associés à un traumatisme rachidien et/ou une lésion crânienne, du syndrome du côlon irritable et de l'obésité.

10. Utilisation d'un composé de formule (I) ou d'un de ses sels pharmaceutiquement acceptables suivant l'une quelconque des revendications 1 à 5, dans la production d'un médicament destiné au traitement ou à la prophylaxie de la dépression, de l'anxiété, de la maladie d'Alzheimer, du déclin cognitif dû à l'âge, du ADHD, de l'obésité, de l'altération cognitive faible, de la schizophrénie, de déficits cognitifs dans la schizophrénie et d'un ictus.

11. Composition pharmaceutique comprenant un composé de formule (I) ou un de ses sels pharmaceutiquement acceptables suivant l'une quelconque des revendications 1 à 5, destinée à être utilisée dans le traitement de l'anxiété, de la dépression, de l'épilepsie, de troubles obsessionnels compulsifs, de la migraine, de troubles cognitifs de la mémoire, de la maladie de Parkinson, du ADHD (trouble de déficit d'attention/syndrome d'hyperactivité), de troubles du sommeil, de troubles de l'alimentation, d'attaques de panique, du sevrage après l'abus de substances médicamenteuses, de la schizophrénie, d'un ictus et également de troubles associés à un traumatisme rachidien et/ou une lésion crânienne, du syndrome du côlon irritable et de l'obésité.

12. Composition pharmaceutique comprenant un composé de formule (I) ou un de ses sels pharmaceutiquement acceptables suivant l'une quelconque des revendications 1 à 5, destinée à être utilisée dans le traitement de la dépression, de l'anxiété, de la maladie d'Alzheimer, du déclin cognitif dû à l'âge, du ADHD, de l'obésité, de l'altération cognitive faible, de la schizophrénie, des déficits cognitifs dans la schizophrénie et d'un ictus.
